# EUROPEAN PATENT APPLICATION

(11) **EP 1 038 792 A1**
(43) Date of publication of application: **27.09.2000**
(21) Application number: 99870051.2
(22) Date of filing: 19.03.1999
(51) Int. Cl.: B65D 75/58, B65D 81/32

(54) **Improved packaged article**

(71) Applicant: THE PROCTER & GAMBLE COMPANY, Cincinnati, Ohio 45202 (US)
(72) Inventor: Crozet, Yvon, I-00168 Roma (BE); Rogers, Neil John, 1020 Brussels (BE); Deflander, Joseph Fernand, 3150 Wespelaar (BE)
(74) Representative: Engisch, Gautier

(57) **Abstract**

The present invention is directed to a packaged article comprising at least one article (1) and an openable outer package (18), the article comprising a cell system with at least one burstable cell (11) filled with an active composition, the packaged article being characterized in that the outer package is made out of material which has a water vapor transmission rate (WVTR) of less than 6g/sqm/day at 40°C/90%RH. Optionally also, the film has an oxygen transmission rate (O₂ TR) of less than 200cc/sqm/day/atm at 23°C/50%RH. Preferably, the cell system is associated with a dispersing element, such as for example a fibrous pad, or cotton pad. Preferably, the force required to burst the cells is comprised within the range 10 to 80 N. In one most preferred embodiment, the cells cannot be refilled and so the article is a one-use disposable article.

## Description

### Field of the invention

The present invention relates to packaged articles comprising one or several cells filled with an active composition, to which an absorbent substrate is preferably associated which contacts the cells contents at the time said cells are being opened.

### Background of the invention

Such packaged articles comprising at least one capsule filled with an active composition, preferably associated to a substrate, for example a cotton pad are representative of the various articles to which the present invention can apply; such articles typically comprise at least one capsule filled with an active composition, and at least one element made out of an dispersing material. At the time the user breaks the capsules, the contents spread onto the dispersing material and the article is ready to use.

The following references are directed to such articles comprising a capsule system associated to an absorbent substrate: *US 4.878.775* (D1) is a US patent assigned to 3M. It discloses a device comprising burstable microcapsules containing a liquid, at least one sheet of a carrying substrate, and at least one sheet of a flexible liquid permeable material overlaying the microcapsules. Pressure on the device breaks the microcapsules and the liquid is dispensed on/through the liquid permeable sheet. *US 3.768.916* (D2) is a US patent assigned to Medical Supply C^{y}. It discloses a sponge comprising a hollow portion into which a glass ampoule is inserted. When the ampoule is broken by the user, the contained disinfectant soap impregnates the sponge for wound-cleaning use. *US 5.090.832* (D3) is a US patent assigned to Colgate. It discloses a disposable pad comprising a scrubber layer, a blotter layer of absorptive material and a liquid impervious sheet located between the scrubber and the blotter layers. The pad further comprises at least one packet containing sufficient cleaning material to saturate the scrubber layer when ruptured. *FR 2.754.744* (D4) is a French patent application to A, Soares Duarte. It discloses a flexible film comprising cells filled with liquid. The cells are ruptured when pressure is exerted and the contained liquid is released and impregnates a support. *DE 3.545.926 A1* (D5) is a German application to A.G. Frühauf. It discloses a sheet-like material comprising capsules filled with an active ingredient, and an impervious separation layer. The capsules have predetermined breaking points or weak points in such arrangement as to orientate the flow of ingredients coming outward when the capsules are ruptured. *EP 0 294 189 A2* (D6) is a German patent application to Jaypak Ltd. It discloses a flexible bag combined to an absorbent applicator. The bag is burst to release the liquid inside the applicator.

While solving several issues, the above mentioned inventions still have some disadvantages. D1 discloses an article comprising microcapsules filled with a liquid composition. The size of the capsules is less than 3mm in diameter because of the material they are made of. This limits the range of uses because the volume of contained liquid is limited.

Some of the inventions, such as the one disclosed for example in D2, comprise a capsule filled with an active composition which is made out of glass. While such capsules can contain large amounts of active composition, they are made out of glass, or similar rigid materials, which become dangerous for the user once they are broken.

Documents D3 to D6 disclose articles wherein the capsules are not limited in volume, and are made out of a material which breaks without a risk of injury for the user - in most cases, the material is a substantially flexible film.

However, for certain tasks the capsules or cells need to be filled with an active composition which is sensitive to gases, vapors, or light. While some documents refer to articles which comprise at least one liquid impervious element, none of the cited art raises the problem of degradation of the capsule's contents by exchange of vapors/gases between the interior and the exterior of the capsules.

It is therefore one main object of the present invention to provide the user with an article comprising at least one cell filled with an active composition and a substrate to be in contact with the cell's contents once said cell has been broken, the cell being made of a material which is such that the size of the cells is not limited to less than 3 mm in diameter.

It is another object of the present invention to provide the user with an article wherein the cell presents no risk of injury to the user, once it was broken.

It is a further object of the present invention to provide the user with an article wherein the force for rupturing the cell is easily applicable by any user, while preventing accidental rupture during manufacture, transportation and storage.

It is another object of this invention to provide the user with an article which prevents substantial exchanges of gases or vapors between the exterior of the article and the interior of the cell.

### Summary of the invention

The present invention is directed to a packaged article comprising at least one article and an openable outer package, the article comprising a cell system with at least one burstable cell filled with an active composition, the packaged article being characterized in that the outer package is made out of material which has a water vapor transmission rate (WVTR) of less than 6g/sqm/day at 40°C/90%RH. Optionally also, the film has an oxygen transmission rate (O₂TR) of less than 200cc/sqm/day/atm at 23°C/50%RH. Preferably, the cell system is associated with a dispersing element, such as for example a fibrous pad, or cotton pad. Preferably, the force required to burst the cells is comprised within the range 10 to 80 N. In one most preferred embodiment, the cells cannot be refilled and so the article is a one-use disposable article.

In one embodiment of the invention, the article comprises a backing sheet which is made out of a high barrier film, as previously defined in terms of MVTR and/or O₂TR properties, said article being folded in half, and two halves being sealed together along their peripheral edges so that said backing sheet forms a unitary outer package, and the shape of the article is a clam-shell.

### Brief description of the drawings

The invention will now be explained in detail with reference to the accompanying drawings, in which:
- Figure 1 is a perspective view showing a cell system comprising several cells, which is integrated in a dispersing fibrous pad.
- Figure 2 is a perspective view of an article in half-folded position with one cell integrated into the dispersing pad, and means to hold the article in the closed position.
- Figure 3 is a profile cut view showing several articles packed inside a separate outer barrier package.
- Figure 4 is a perspective view showing a article with an outer barrier package integrated as a backing sheet, for individual liquid/vapor/gas barrier properties.
- Figure 5 is a profile cut view showing one cell comprising a flow-control means with cut-out portion.
- Figure 6 is a profile cut view showing one cell comprising a flow-control means with centered cut-out portion and attached flow-control window.
- Figure 7a is a profile cut view showing one cell comprising a flow-control means with off-centered cut-out portion and attached flow-control window. Figure 7b is similar to Figure 7a but shows the article in folded position.
- Figure 8 is a profile schematic view showing one cell of the cell system formed as a sachet.
- Figure 9 is a profile schematic view showing one cell of the cell system with integral flow-control means.

### Detailed description of the invention

The article of the present invention has one or multiple enclosures or closed cells which can contain fluid or solid (including powdered) products, preferably liquid products, which are released from the individually sealed enclosures via the increase of internal pressure within the enclosure eventually leading to rupture of one of the enclosing materials or a seal that may be present between two pieces of enclosing material. As alternatives to compression, a gas-producing reaction can be initiated to increase the cell's internal pressure to the point of rupture, or snapping, chemically dissolving or puncturing operation can be used.

The invention is more generally related to a single or multiple cell system connected by some means to create an article or substrate of a system of chamber(s) or cell(s). The cells are ruptured by one means or another thus opening the chambers to exposure to: (i) the outside environment; (ii) an internal constrained (but larger volume) environment within the system or article; or, (iii) other fluids (e.g. gases) outside of the original chambers which may enter into the previously sealed chambers. The purpose of such exposure is to provide a useful benefit to the user including but not limited to: dispensing fluids or solids (including powders) in the cells to contact and optionally disperse onto a target surface for a variety of purposes ; mixing different materials from different cells to cause a useful chemical reaction as described above ; exposing a reactive material to a fluid (e.g. gaseous air or liquid water) found in the environment to cause a useful chemical reaction where either the chemical composition or even the phase state of the end products of the reaction or the energy/heat flow of the reaction may be the useful endpoint sought ; exposing a fluid or solid to the gaseous environment for the purpose of volatilizing and distributing components of said fluids or solids.

The above-mentioned applications for the present invention can be directed to a multitude of user-beneficial outcomes including, but not limited to: cleaning, bleaching, cooling, heating, deodorizing, disinfecting, medicating, wiping...

The article of this invention optionally but preferably features a support dispersing material designed to absorb the product upon release. This support material is designed to assist the user in the application of the product.

For the article, barrier properties are required to prevent significant product(s) loss through permeation (i.e. less than 10 % product loss/year at 35°C/20% RH and the product(s) must keep the activity it is designed for). The barrier properties of the closed cell(s) level are not controlled, but instead, the barrier properties are provided by a high barrier secondary structure covering the entire article.

### The cells

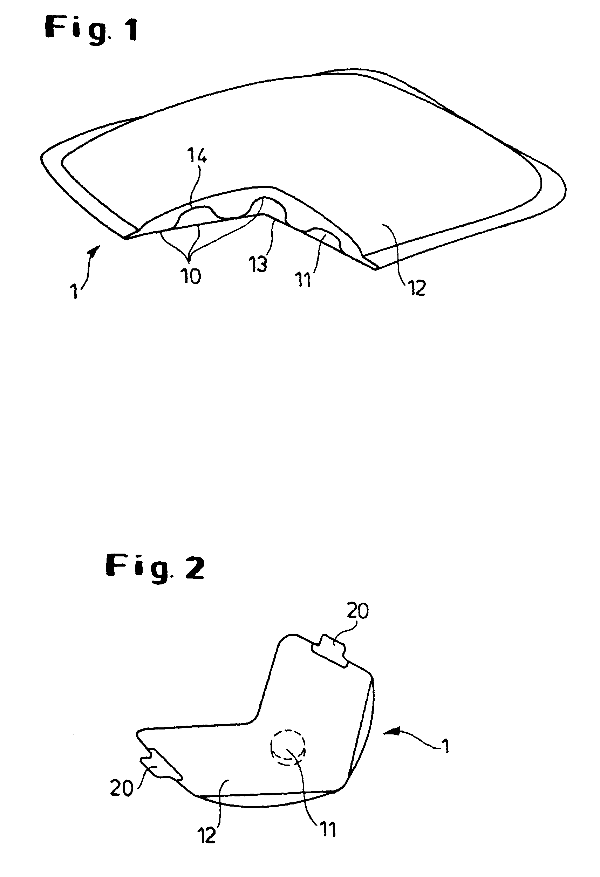

As shown in figures 1 to 7, the article (1) is provided with a cell system (10) which comprises at least one cell (11) filled with an active composition, which can be either solid, particles, granulates, powder, liquid, or even a gas, but is preferably a liquid composition. Depending on the purposes, the cell system (10) can comprise one or more cells, filled with the same, or different compositions (see examples of contained compositions below). The cell system (10) can be used by itself, or it can also be coupled to a solid medium, for example an absorbent support (12). It can also be integrated into a pouch filled with liquid or gas.

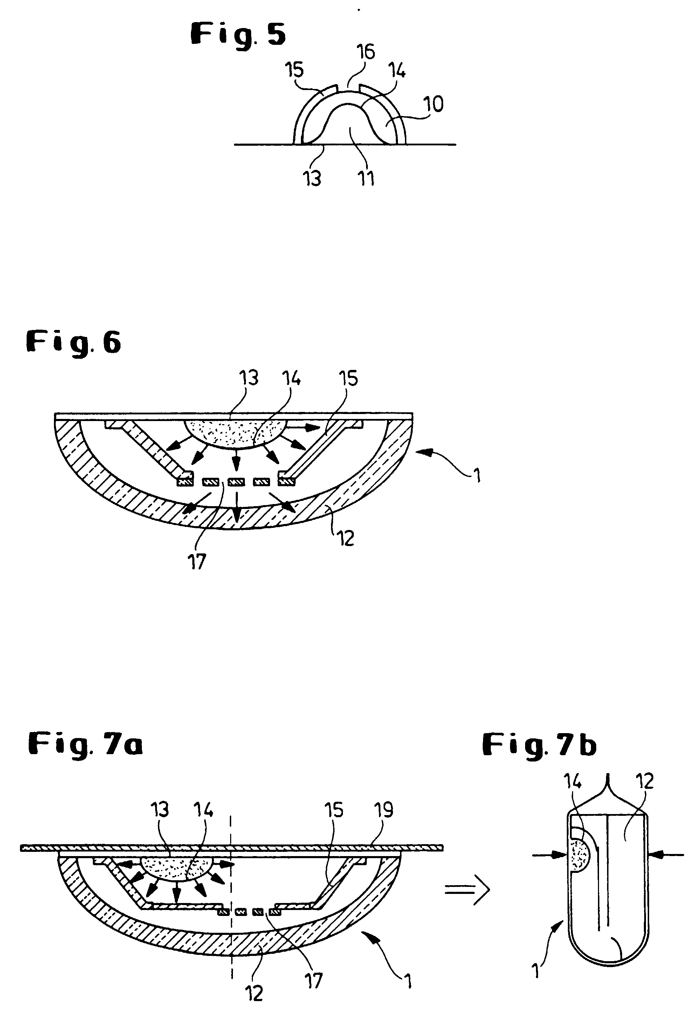

One cell (11) comprises a bottom (13) and a dome (14) . The dome (14) can be made out of a flat film which is deformed into the cavities of a mold, for example by a thermoforming process. Once filled with the active composition, the bottom (13) - which is a flat film - is closed by sealing the bottom (13) which preferably is a flat film. The cells (11) are ruptured by applying a constraint onto their surface which can be mechanical such as a pressure, a tearing or peeling movement; or a chemical reaction which dissolves the surface of the cell. In one preferred embodiment, the cells (11) are burst when the user applies a compression force on the top or the sides of the cell, as shown for example in figure 7b. This leads to rupture one portion of the dome (14) of the cell (11). The cell (11) normally ruptures in its dome portion (14). This is due either to the fact that the material used to make the cell bottom is chosen as more resistant to rupture than the material for the dome (14), or in case the same materials are used for forming the bottom (13) and the dome (14), this is due to the manufacturing process: since the dome (14) is thermoformed in this example, its constitutive material thins down in at least one point, where mechanical properties, and especially resistance to burst, are decreased. In another embodiment , the cells (14) are chemically dissolved.

The cells (11) can have any shapes or dimensions, such as for example parallelepipedic, oval or hemispheric, but while not being limited, their volume is preferably comprised within the range of 0.01 to 5.0 ml, more preferably within the range of 0.4 to 2.0 ml. For example in the case the cells are substantially hemispheric, their diameter is preferably comprised within the range of 10 to 20 mm and their depth is between 6 and 15 mm. Preferably, 50 to 99% of one cell's volume is filled with liquid. In the case of cells filled with a liquid active composition, the cell (11) is preferably made out of a liquid impervious material.

While the cell bottom and top can be joined or sealed by a variety of known techniques including adhesives, the cell's material should preferably feature good sealing properties and mechanical properties such that the user can rupture the cells to get the contained active composition out by applying a reasonable force, while the cells need to be strong enough not to rupture prematurely, for example during manufacturing, storage or transportation. Furthermore, the material shall be chosen such that it does not produce sharp particles once broken, especially glass shall not be used since it produces glass splinters which can be very dangerous for example when the article (1) is used for facial/body cleaning purposes.

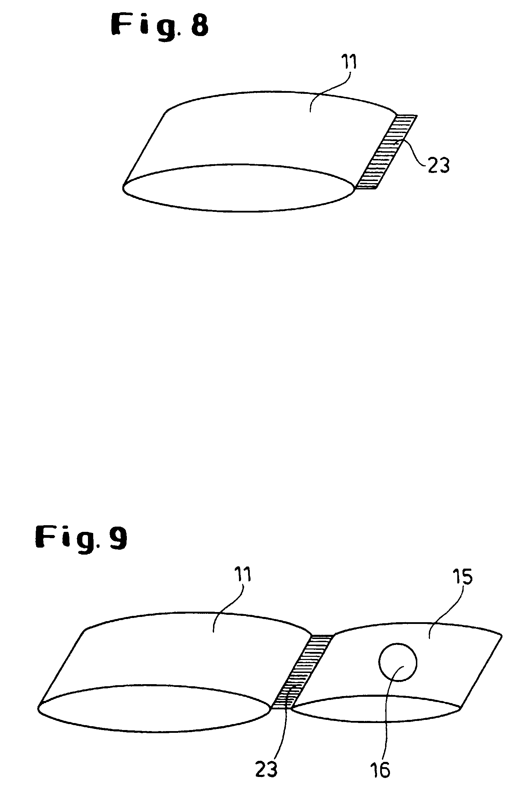

In another embodiment, the cells (11) have the shape of sachets, as shown for example in figure 8. Such sachets are made out of a film which is folded and sealed. Preferably, the sachet is made of a film which is easily rupturable, or alternatively, it comprises at least one seal (23) which is peelable, and opens when the user applies a reasonable pressure on the sachet, as described hereafter.

The force to be applied to one cell to burst it, is substantially comprised within the range of 10 to 80 N (force applied on top of the cell). Preferably, the force to burst the cells (11) is less than 60 N, more preferably less than 40 N. Most preferably it is comprised within the range of 10 to 20 N. It has been found that this force to burst usually decreases for a same cell when the force is applied on the sides for substantially hemispheric shaped cells. The material is preferably a thermoplastic, such as polypropylene or polyethylene. More preferably, said material is a low density polyethylene film (LDPE film) which features good processability, especially when using a thermoforming process. In any case, it has been found that the preferred rupture properties for the cell are achieved for a film with limited thickness - typically less than 90µm. This however leads to low barrier properties: in particular when one of the water vapor transmission rate (WVTR) is higher than 6g/sqm/day at 40°C/90%RH, and also optionally the O₂TR (oxygen transmission rate) is higher than 200cc/sqm/day/atm at 23°C/50%RH, at least some ingredients of some composition may escape from the cell thus leading to a change in the proportions of the composition, and modification of its properties. It has further been found that such changes are rapid enough to appear during the period of time between the manufacture of the cell system, and its use (including transportation and storage periods).

Through not to be limited, the thickness of the material used for making the cells is preferably comprised within the range of 35 to 90 µm, more preferably within the range of 35 to 50 µm. When the thickness is below 35 µm, it was found that manufacturing problems appear, for example wrinkles appear during thermoforming, and the force to rupture the cells is decreased - typically below 10 N - which leads to premature burst. It has also been found that a thickness above 90 µm leads to excessive force required to burst the cells - typically above 100 N -, which is clearly undesirable to the consumer. However there are compensating levers independent of the thickness such as cell size to adjust the rupture force.

For such cells, the loss of contents depends on a variety of parameters such as external temperature, nature of the contained composition (water/alcohol/solvents...), thickness of the cell's dome (14), thickness of the cell's bottom (13), diameter and shape of the bubble. Furthermore, as previously explained, for a given material and volume (shape/size) of the cell, the force to burst the cell depends on the thickness of the material. Acceptable values of the force to burst a cell are achieved for thin films, i.e. films with a thickness preferably below 90 µm, more preferably below 50 µm. However, good burst properties in this case are counterbalanced by poorer barrier properties. This leads to permeation through the cell's material, and modification of the composition and its properties. This issue is solved by providing the articles with an outer package, which is made out of a high barrier material, as hereafter described.

### The support material

Preferably, the cell system (10) is coupled to a support material (12). In a first embodiment of this invention, said support material is made out of a liquid impervious material, such as for example a flexible pouch which contains the cell system. In another embodiment of this invention, the support material is an dispersing material (12), such as for example, a fibrous pad, preferably a cotton pad for facial cleaning, a wipe for body or household cleaning made out of a non-woven material, a pad to be used for wound-protection, as shown for example in figures 1, 2 or 4. Examples of fibrous pads are already known in the art, and some of them are particularly described in US.5.738.212.

Said dispersing material (12) can have any shape, and dimensions suitable for containing or being coupled with a cell system (10) with dimensions as defined above. Once the active composition contained inside the cell (11) has been released, said composition disperses into the structure of the dispersing material, up to its surface. The structure of said dispersing material can be chosen so as to fit the purposes of the article (1): it can have a smooth surface for pampering, or a rough surface for cleaning, rubbing or removing dead skin for example, or it can be spongy for moisturizing/impregnating the surface to treat. The thickness, shape and dimensions of the dispersing material should be chosen relative to the number of cells (11) comprised inside the cell system (10), and the volume of active composition contained inside each cell (11).

### The flow control means

It has been found that in some occasions, and particularly when the active composition contained inside the cells (11) comprises a liquid or cream form, spillage of said composition can occur at the time the cell (11) is ruptured, even when the cell system (10) is coupled to or associated with an dispersing material (12). To prevent spillage and/or ensure good absorption and diffusion of the composition at the surface of the dispersing material (12), if any, the article (1) of the present invention is optionally but preferably provided with a means to control the flow of released active composition when the cell (11) bursts.

As shown in figures 5 to 7, the flow-control means (15) is for these examples, a film which covers at least the dome (14) side of the cell (11) and comprises at least one restricted area for the active composition to pass through, from the cell (11) to the outside medium. Optionally, said film also covers the bottom (13) of the cell (11), so as to prevent any spillage of the composition from the bottom (13) side of the cell (11) in case of accidental or intentional burst of said bottom (13) of the cell (11). In one embodiment, the flow-control means (15) is provided with a cut-out portion (16), through which the active composition released from the cell (11) is directed. In another embodiment, the flow-control means (15) is achieved by a cut-out portion of the film onto which a flow-control window (17) such as for example a grid, or a sheet of a porous material is adapted, as shown in figures 6 and 7. Such a grid material is described for example in US.3.929.135 to Thompson, or in US.4.324.246 to Mullane, or in US.4.342.314 to Radel. Another grid material which can be used is known under the trademark name "DRI-WEAVE™" marketed by The Procter & Gamble Company.

Preferably in both of the preceding embodiments, and as shown in figure 7, the cut-out portion (16) or the flow-control window (17) is not aligned with a cell (11), so that the way of released composition from the cell to the exterior - for example, to the absorbent cotton pad (12) - is diverted. This allows to better prevent spillage of composition to the exterior, especially in case said composition has a very low viscosity (i.e. less than 100 cps measured with a viscosity meter at 20°C, 1200 rpm).

Alternatively, when the cell has the shape of sachet (11) made of a film which is folded in halves, with sealings on the sides, the flow control means (15) can be an extension (24) of the sachet which comprises for example a punched portion (16), as shown in figure 9. The main sachet-cell (11) which is filled with a product, and its flow-control extension (24) are separated by a peelable seal (23). Both are manufactured from the same film which is folded in two halves and sealed, with one median seal (23), so that when the user applies a pressure on the top of the filled portion of the sachet (11), it breaks the peelable seal (23), creating a channel between the sachet (11) and the flow-control extension (24), and the product is released through the punched portion (16).

### The outer package

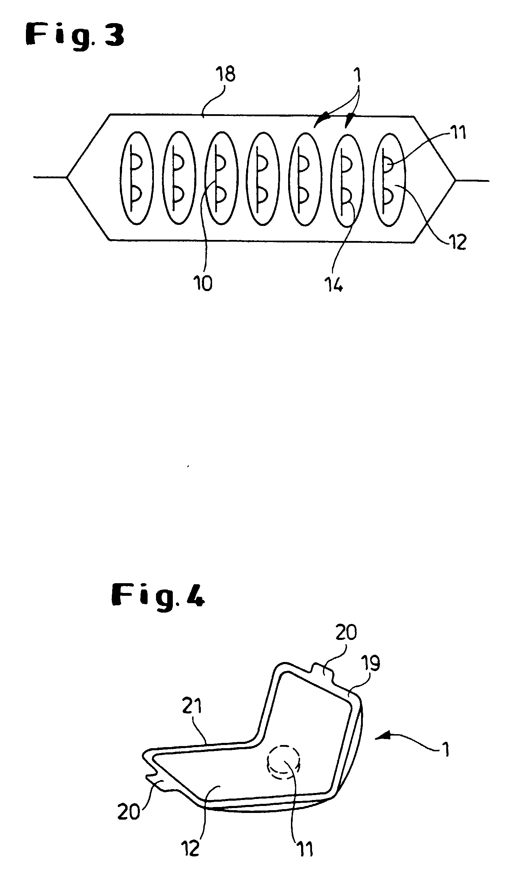

An outer package is provided which can be of any type suitable for containing at least one article (1). Said outer package can be a separate element (18) (see figure 3), for example a wrap-around film which surrounds at least one article (1), a laminated carton, a glass or plastic jar. Alternatively, it is an element integrated (19) to the article (1) itself, as shown in figure 4. As explained above, the material used for the cell (11) is chosen such that the cell (11) shows a low burst force but this leads to poor barrier properties, and thus modifications of the contained active composition by permeation of some components of the composition through the cell (11). Said outer package is defined as being made out of a barrier material. Such a material is defined as follows: it is liquid impervious in that no liquid passes through it after 30 sec.; it is a barrier to vapors/solvents in that its water vapor transmission rate (WVTR) is less than 6g/sqm/day at 40°C/90%RH; also optionally it is a barrier to gases in that its O₂TR (oxygen transmission rate) is less than 200cc/sqm/day/atm at 23°C/50%RH.

In one embodiment shown in figure 3, the outer package (18) is a film which is folded and sealed around at least one article (1), for example by using a wrap-around or wrapping process. The film (18) can have one thick layer of a thermoplastic, but is preferably a laminate material comprising at least one layer of a barrier material such as an aluminium laminate for example, which is sandwiched between thermoplastic outer layers, for good sealing properties of the film. In a most preferred embodiment, the film (18) is laminated comprising at least one aluminium layer which gives very good barrier properties to liquids, gas and vapors, for example a LDPE/Alu/PET (Low Density Polyethylene/ Aluminium/ Polyethylenetherephtalate having a thickness of respectively 80µm/9µm/12µm. Such a film has a permeation of less than 10% product loss/year at 35°C/20%RH, so that the active composition keeps the activity it has been designed for. It can also be a LDPE/metallized bi-oriented PP/PET laminate, or a LDPE/metallized bi-oriented PET/PET laminate.

In another embodiment shown in figure 4, the outer package (19) is designed as an integral part of the article (1), which is attached to the other elements, for example the cell system (10) or the absorbent support (12) material. The film material which is used should feature barrier properties to liquid, gas, and vapors, as previously defined for the separate outer package, so as to obtain a permeation of less than 10% product loss/year at 35°C/20%RH. For example, and as shown in figure 4, the film (19) is integrated as a back-sheet of the article. Preferably, as shown in figures 7a and 7b, the surface of said barrier backing sheet is greater than the surface of the absorbent pad, so that once folded, the peripheral portions of the backing sheet are sealed to close the article as a packaging unit (see figure 7b).

### Examples

Different possible embodiments of articles according to the present invention will now be described in detail, with reference to the accompanying figures.

In a first embodiment of the present invention, one article (1) comprises a cell system (10) with at least one cell filled with an active composition. The cell is covered by a flow control means, such as a film with a cut-out portion (16) which is off-centered relatively to the dome (14) of the cell. The flow-control film is attached onto the cell system (10) by sealing or gluing its edges to the cell system's edges. Said cell system (10) is further sandwiched between two layers of an dispersing material (12) such as cotton, so as to create an absorbent cotton pad with an integrated burstable cell where the two outer layers of cotton can be glued or taped one to the other, but are preferably sealed, using the thermoplastic material of the cell system (10) and/or flow-control means (15) as a sealing agent. The cotton pad further comprises an integrated outer package with barrier properties to liquids, gases and vapors, and permeation properties as defined above. Said outer package (19) is integrated to the article (1) as a backing sheet which is glued or sealed to the outer side of the pad opposite to the dome (14) of the cell, as shown in figure 7. Preferably, the surface of the barrier backing sheet is slightly superior to the surface of the cotton pad, so that when the pad is folded into two halves into a clamshell-like article, both halves being maintained in closed position by a peelable peripheral seal (21), the backing sheet protects the whole cotton pad inside from the outside medium. The clamshell article (1) is used by peeling or cutting the outer package open, bursting the cell to release the active composition into the cotton pad, and then using it. Preferably, the cell is off-centered in the pad, so that it is easier to burst by squeezing the two halves of the pad with one hand by pressing onto the outer package, as shown in figure 7b (black arrows). The clam-shell structure enables the consumer to use a pad whose surface which comes in contact with the surface to treat is never in contact with another surface (i.e. fingers of the user or neck of a bottle), and thus is very clean, even once the active composition impregnates the pad. This renders this structure particularly useful for cosmetic purposes.

In a second embodiment of the invention, and as shown in figures 2 and 3, each article (1) comprises a cell system (10) with at least one cell (11) filled with the active composition. It further comprises an absorbent pad (12), for example a cotton pad which contains the cell system (10). The pad is folded into two halves which are maintained by portions (20), which are glued, peel-sealed, taped, or maintained by another suitable means for securing the pad in folded position while being easily openable during use. The barrier to liquids, gases or vapors is achieved by a separate outer package which, in this example, is a laminate aluminium film wrapped around at least one article (1), as shown in figure 3. To use the pads, the user opens the outer package, bursts the cell(s) into the pad, unfolds the pad and uses it.

In another embodiment, each article (1) comprises a cell system (10) with at least one cell (11) filled with the active composition. It further comprises an absorbent pad (12), for example a cotton pad which contains the cell system (10). The pad is folded into two halves which are maintained by portions (20), which are glued, peel-sealed, or maintained by another suitable means for securing the pad in folded position while being easily openable during use. The barrier to liquids, gases or vapors is achieved by a separate outer package which is a carton outer box. More specifically, said box is made out of a laminated carton, i.e. the carton comprises at least two layers attached one to the other(s) for example by glue: the outer layer is a flat carton, for example a folding box board, the inner layer is a barrier layer made out of Aluminium. To use the pads, the user opens the outer package, bursts the cell(s) into the pad, unfolds the pad and uses it.

### Contents

The active composition which is contained inside the cell can be of any type, for example a face cleaning lotion or cream, a disinfectant composition, a composition for household cleaning purposes, a perfume composition, a polish, a medicament... Its viscosity is preferably comprised within the range of 0.1 to 6000cps measured at 20°C. In one embodiment of the present invention, the active composition is a liquid face cleaning and disinfecting composition known under the trademark Clearasil™ manufactured and sold by The Procter & Gamble Company. Such a composition typically comprises for example: Purified Water; Alcohol 96%; Diethyl Phthalate; Myrtrimonium Bromide; Ceteareth-14; Disodium Cocoamphodiacetate 38%; Tartaric Acid; Allantoin; Chlorhexidine Digluconate 20%; Perfume Balance PSV 1270; Cl 42051 Acid Blue 3 E131. Detailed examples of such composition are disclosed for example in European Patent Applications EP-A-0614353 and EP-A-0614354 to the Procter & Gamble Company.

## Claims

1. A packaged article comprising at least one article (1) and an openable outer package, the article (1) comprising a cell system (10) with at least one burstable cell (11) filled with an active composition, the packaged article being characterized in that the outer package is made out of material which has a water vapor transmission rate (WVTR) of less than 6g/sqm/day at 40°C/90%RH.

2. A packaged article according to any of the preceding claims, wherein the force required to burst one cell (11) is comprised within the range of 10 to 80 N, preferably 10 to 60 N, more preferably 10 to 20 N.

3. A packaged article according to any of the preceding claims, wherein the cell system (10) is coupled to a dispersing article for controlling the application of said active composition once said at least one cell (11) has been burst.

4. A packaged article according to any of the preceding claims, wherein said dispersing article (12) is a fibrous pad.

5. A packaged article according to any of the preceding claims, wherein each article (1) comprises a backing sheet (19) which is made out of a material which is a high barrier film, said article being folded in half and two halves being attached together by peripheral seals, so that said backing sheet forms said outer package.

6. A packaged article according to claim 5, wherein, the peripheral seals are peelable seals.

7. A packaged article according to any of the preceding claims, wherein said outer package is a LDPE/Alu/PET film laminate, or a LDPE/metallized bi-oriented PP/PET laminate, or a LDPE/metallized bi-oriented PET/PET laminate.

8. A packaged article according to claims 1 to 6, wherein said outer package is a laminated flat carton comprising an aluminium inner layer, or metallized layer.

9. A packaged article according to any of the preceding claims wherein the cells (11) have an internal volume which is comprised within the range of 0.01 ml to 5.0 ml, preferably 0.4 to 2.0 ml.

10. A packaged article according to any of the preceding claims, wherein 50 to 99% of one cell's volume is filled with liquid.
